Europäisches Patentamt

European Patent Office

Office européen des brevets

(11) Publication number: **0 660 104 A2**

## EUROPEAN PATENT APPLICATION

(21) Application number: **94120700.3**

(22) Date of filing: **27.12.94**

(51) Int. Cl.6: **G01N 15/14**

(30) Priority: **27.12.93 JP 333264/93**
**09.03.94 JP 38742/94**

(43) Date of publication of application:
**28.06.95 Bulletin 95/26**

(84) Designated Contracting States:
**DE IT**

(71) Applicant: **HITACHI, LTD.**
**6, Kanda Surugadai 4-chome**
**Chiyoda-ku,**
**Tokyo 101 (JP)**

(72) Inventor: **Yabe, Ryohei**
**4829-28, Nakane**
**Hitachinaka-shi,**
**Ibaraki 312 (JP)**
Inventor: **Kurimura, Masaaki**
**560-4, Sugi,**
**Naka-machi**
**Naka-gun,**
**Ibaraki 311-01 (JP)**
Inventor: **Asai, Hideki**
**19, Kitami-cho 4-chome**
**Mito-shi,**
**Ibaraki 312 (JP)**
Inventor: **Kojima, Yasuaki**
**1108-7, Sawa**

**Hitachinaka-shi,**
**Ibaraki 312 (JP)**
Inventor: **Sano, Kazuhiro**
**Takenouchi Haitsu 202,**
**2694-1, Sugaya**
**Naka-machi,**
**Naka-gun,**
**Ibaraki 311-01 (JP)**
Inventor: **Oowada, Norio**
**Tsukubadai Apartment 6-102,**
**663, Ichige**
**Hitachinaka-shi,**
**Ibaraki 312 (JP)**
Inventor: **Katahira, Hatsue**
**5377-9, Sugaya,**
**Naka-machi**
**Naka-gun,**
**Ibaraki 311-01 (JP)**

(74) Representative: **Altenburg, Udo, Dipl.-Phys. et**
**al**
**Patent- und Rechtsanwälte,**
**Bardehle . Pagenberg . Dost . Altenburg .**
**Frohwitter . Geissler &**
**Partner,**
**Galileiplatz 1**
**D-81679 München (DE)**

(54) Urinary sediment examining apparatus.

(57) A urinary sample is conducted in a flow cell, and particles are detected so as to pick up images thereof in response to the detection of the particles and are classified on the basis of the detected images. An operator judges whether automatic classification is made or not. Particles of the particle item to be automatically classified are automatically classified by designating the particle item. With respect to particle items to be manual-data-corrected, object fields of view corresponding thereto are registered in advance. Therefore, when a particle item to be manual-data-corrected is designated, data on particles of the designated particle item for the field of view corresponding thereto are produced.

Rank Xerox (UK) Business Services
(3.10/3.09/3.3.4)

EP 0 660 104 A2

# FIG. 1

The present invention relates to a urinary sediment examining apparatus, and more particularly to a urinary sediment examining apparatus suitable for classifying particles suspended in a urinary sample.

When a technical expert of examination visually examines particles in urine, the following processes are required.

(1) Sampling some urine in a test tube and separating particles in the urine by centrifugal separation.

(2) Dripping the precipitated particles on a slide glass and staining the same if necessary.

(3) Preparing a slide sample and observing it using a microscope.

(4) Counting the number of abnormal particles in predetermined several regions.

The observation power is switched over according to particle items to be classified. In reporting the results of examination, they need to expressed so as to include the number of particles in a high power field of view, the number of particles in a low power field of view and the number of particles in a whole field of view according to particle items as described in "URINARY SEDIMENT EXAMINATION METHOD" published by The Association of Japan Clinical and Medical Examination Technologists, August 14, 1991.

With regard to apparatuses for automatically performing the above processes, there is an apparatus in which the particles are optically analyzed by conducting a liquid including the particles suspended therein through a flow cell. For example, as disclosed in Japanese patent publication No. 52573/1991, there is an apparatus in which a liquid sample is conducted through a flow passage having a special shape and therein optical still images of the particles in the sample are produced. According to this technology, morphological features of the particles can be extracted by converting the optical still images into electronic images to analyze them, and hence the particles in urine can be classified on the basis of the extracted morphological features. Further, as disclosed in Japanese patent application No. 38653/1985, the particles can be sub-classified into classes of visual recognition characteristics on the basis of the morphological analysis of the particles using the optical still images. For example, the particles can be classified in detail according to a specified order such as sizes, colors or the like. In the urinary sediment examination, the classification work of the technical expert of examination is partially automated by conducting the urine through the flow cell to produce the still images of the particles and to convert the same into electronic images.

Since the urine of healthy person has very small amount of particles in urine, the classification and the counting of particles are comparatively easily performed by the visual classification of the technical expert of examination. However, morbid urine has high concentrations of amorphous saline, mucus, erythrocytes, leucocytes and so on, and contains extremely many particles. Although automation of an examining apparatus is effective for the urine containing a lot of particles, there are the following problems.

(1) When the particles are classified according to order of sizes, both erythrocytes and leucocytes in urine are fell in the same class together since their sizes are overlapped each other.

(2) When the particles are classified by color, hyaline casts and mucus are fell in the same class together since their colors resemble each other.

In order to solve these problems, a technical expert of examination classifies the mixed particles with visually judging using optical still images produced by an examining apparatus.

Since the urine of morbid person has many kinds of particles, it takes long time and becomes unpleasant work for the technical expert to judging all the particles, which leads to an inaccurate result. Further, there is a possibility to miss the particles having an important diagnostic meaning but a small frequency of occurrence (for example, atypical cells, cancer cells and so on).

Since the conventional method is, as described above, insufficient in judgement of classification, inferior in efficiency and improper in accuracy, there is a disadvantage in that a lot of particles cannot be classified and counted efficiently and speedy urinary sediment examination cannot be performed.

There are, as described above, generally various kinds of particles in urine and hence it is impossible to automatically classify all the particles. Therefore, it is practical that only particle items having high frequency of occurrence are automatically classified and the remaining particle items are precisely classified by a technical expert to obtain a final examining result. For automatic measurement in the urinary sediment examination, it is necessary to calculate the number of particles per unit volume using the volume of urine used in measurement and the number of particles measured.

However, either of the aforementioned references pays no special consideration to efficiently provide accurate examination data on the particle item predetermined to need manual data correction manually designated by a technical expert or an operator when there are particles which cannot be classified automatically or when an automatically classified result needs to be precisely classified further.

An object of the present invention is to provide a urine sediment examining apparatus suitable for automatically classifying particles suspended in a liquid sample with high accuracy and high efficiency.

Another object of the present invention is to provide a urinary sediment examination apparatus suitable for efficiently obtaining accurate examination data on particle items predetermined to need manual data correction manually designated by an operator when the particles cannot be classified automatically or when the automatically classified result needs to be precisely classified further.

According to the present invention, a urinary sediment examining apparatus is provided which comprising means for passing through a predetermined position a liquid sample in which particles are suspended, means for detecting a particle passed through the predetermined position, means for designating an particle item predetermined to need automatic classification, and means for producing an image of the detected particle and determining whether the detected particle is to be classified as the designated particle item on the basis of the image.

According to such a urinary sediment examining apparatus, a technical expert of examination or operator can exclude the particles adversely affecting the classification accuracy thereof and the particles inefficient to classification from the objects for automatic classification and can select the other particles as the objects for automatic classification, and hence the particles selected as the objects for automatic classification in such a manner can be performed automatic classification with high accuracy and high efficiency.

According to another aspect of the present invention, there is provided a urinary sediment examining apparatus having means for detecting particles suspended in a urinary sample, means for producing images of the detected particles and means for classifying the detected particles on the basis of the images, the improvement comprising means for registering object fields of view predetermined corresponding to particle items predetermined to need manual data correction in advance, and means for designating at least one of the particle items, the particle classifying means being adapted to produce data of the detected particles classified as the designated particle item, the data being for the registered object field of view corresponding to the designated particle item.

For particles suspended in a urinary sample which are unable to be automatically classified or particles suspended in the urinary sample requiring further subclassification, an operator is required to observe images of those particles and input the particle item names thereof. In order to express the data of the particle item names as examination results, it is necessary to express the data with special expression methods which are different from one another depending on the particle items and fields of view, i. e., a high power field of view, a low power field of view or a whole field of view.

The measured volume of the urinary sample in the low power field of view is generally 10 or more times that in the high power field of view. Therefore, the number of particles per unit measured urinary sample volume appearing only in the high power field of view is required to be calculated on the basis of the measured volume of the urinary sample in the high power field of view since, if it is calculated on the basis of the sum of the measured volumes of the urinary sample in both high and low power fields of view, it calculated less than one tenth of the actual number.

The present invention is capable of solving such a problem, since the data of the detected particles classified as the designated particle item are for the registered object field of view corresponding to the designated particle item.

Other objects and features of the present invention will become apparent from the descriptions of preferred embodiments of the present invention taken in connection with the accompany drawings in which:

FIG.1 is a schematic view showing the total construction of an embodiment of a urinary sediment examining apparatus according to the present invention,

FIG.2 is a view showing an embodiment of a screen for setting the automatic classification according to the present invention,

FIG.3(A) is a view explaining an example of judging the condition whether automatic classification is required or not according to the present invention,

FIG.3(B) is another view explaining an example of judging the condition whether automatic classification is required or not according to the present invention,

FIG.4 is a flow chart for an example of classification according to the present invention,

FIG.5(A) is a view explaining an example of judging whether automatic classification is required or not using feature parameters according to the present invention,

FIG.5(B) is another view explaining an example of judging whether automatic classification is required or not using feature parameters according to the present invention,

FIG.6 is a view explaining another example of judging whether or not automatic classification is required without counting as designated particle items according to the present invention,

FIG.7(A) is a view explaining a further example of judging whether or not automatic classification is required using weight coefficients according to the present invention,

FIG.7(b) is a flow chart explaining an example of updating weight coefficients in the example shown in FIG.7(A) according to the present invention,

FIG.8 is a view showing an example of a screen for manually setting classification items according to the present invention, and

FIG.9 is a view showing an example of a condition setting screen for particle items predetermined to need manual data correction.

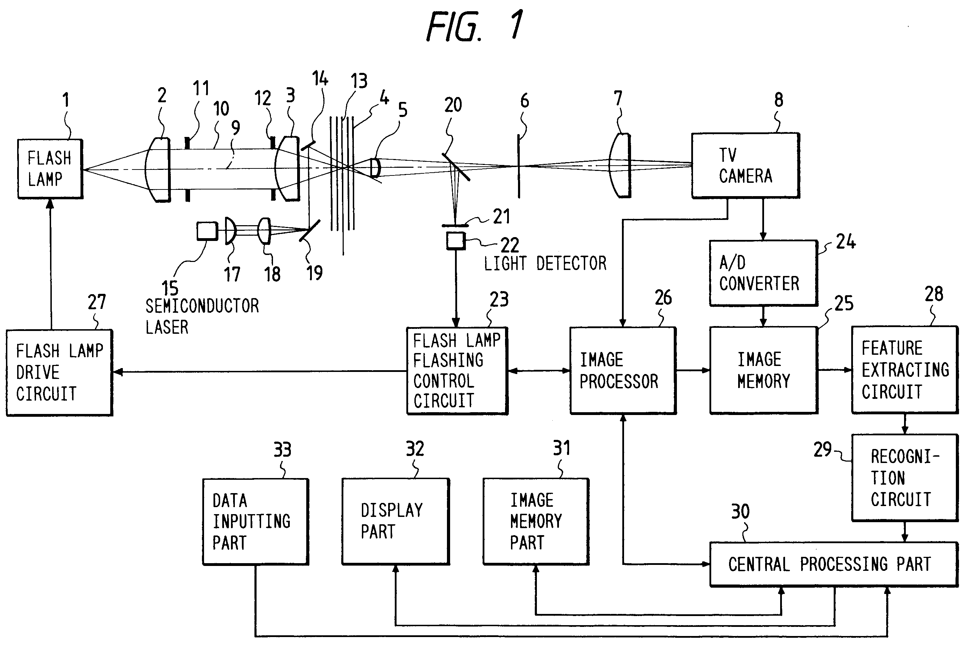

In FIG.1, light emitted from a flash lamp 1 of microscope light source passes along an optical axis 9 of a microscope, converged by a field lens 2, and focused a condenser lens 3 on a liquid sample or urinary sample 13 passed through a flow cell 4. Actually, the light beam 10 from the light source 1 is restricted by a view aperture 11 and an opening aperture 12.

Images of particles suspended in the sample are formed at an image forming position 6 by an objective lens 5 and are focused on an image pickup surface of a TV camera 8 by a focusing lens 7. The TV camera is usually of a CCD type because of low after-image.

Flash timing of the flash lamp 1 is controlled by a detecting signal of a particle detecting system. A light beam from a semiconductor laser 15 continuously oscillating is converted into a parallel light beam by a collimator lens 17, and converged only in one direction by a cylindrical lens 18. The focusing position of the laser beam is at the sample passed through the flow cell, and the laser beam is directed to the focusing position by a reflecting mirror 19 and a small size reflecting mirror 14. When the particles in the sample is passed across the laser beam, this laser light is scattered, and the scattered light is collected by the microscope objective lens 5 used for image pickup of the particle image, and reflected by a beam splitter 20. The reflected scattered light is limited in the observed region in the sample passed through the flow cell by an aperture 21, and converted into an electric signal by a light detector 22 to obtain particle detection signals.

The semiconductor laser 15 always glows to monitor particles in the sample passed through the detecting region any time. When the magnitude of the particle detection signal by the scattered light caused by passing of a particle is larger than a given signal value, the particle is judged as a particle to be image-processed and a flash lamp flashing control circuit 23 controls a flash lamp drive circuit 27 such that the flash lamp 1 glows when the particle comes into a certain position in the image pickup region of the TV camera 8.

Since the glowing period of the flash lamp 1 is set short enough to neglect the moving distance of the particle in the sample during the glowing period, the TV camera 8 can pick up the particle in the sample as a still image when the flash lamp 1 glows. An image processing control circuit 26 performs control to write the image signals after A/D converting into an image memory 25 and the image processing thereafter. In the image processing, a feature extracting circuit 28 extracts primary parameters of the particles such as shape, color, size and so on. The image processing control circuit 26 inputs secondary parameters produced by calculation using the primary parameters and combination of these into a recognition circuit 29. The recognition circuit 29 classifies the particles using a neural network of Rammelhart type. Since the neural network has learned well based on the judgement of experts in advance using a lot of data and the coupling factors among neurons are optimized, automatic classification of particles in the sample can be performed through neural network calculation using the input feature parameters. A central processing part 30 judges from the classified results based on a preset condition whether the image is necessary to be stored and stores the images into an image memory part 31 if the image is needed to be stored.

Further, when necessity for automatic classification for each of particle items is set through input from a data inputting part 33 such as key-board, the central processing part 30 instructs the recognition circuit 29 whether the classification is required or not.

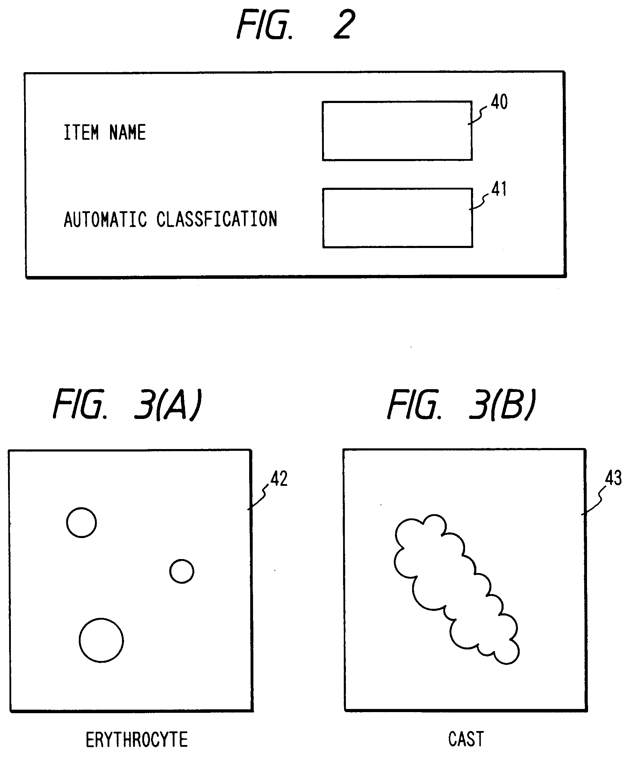

FIG.2 shows a lay-out on the screen instructing input provided to realize the present invention. An particle item name input area 40 is that in which the particle item name to be designated can be displayed. The particle item names displayed, for example, leucocyte, erythrocyte, epithelium, cast, crystal, bacteria, etc, are updated unit by unit according to the input from the key board where one unit is representative of the predetermined number of the particle item names which may be one, two or three. The technical expert of examination sets using the automatic classification designating area 41 whether the automatic classification is required or not. For example, the technical expert of examination inputs "yes" so that it is displayed when the automatic classification is required and inputs "no" so that it is displayed when the automatic classification not required.

The necessity of automatic classification in the above embodiment is determined by judgement of the technical expert of examination. FIG.3 shows an example of judging conditions on whether automatic classification is preformed or not. FIG.3(A) schematically illustrates an image 42 of erythrocytes. FIG.3(B) illustrates an image 43 of a cast. Most of images 42 of erythrocytes are generally observed as torus-shaped or circular particles as shown in FIG.3-(a). In such a case, since relation between the size or area and the length of periphery is nearly constant, it is possible to judge that the detected particle is an erythrocyte. On the other hand, in a case of the image 43 of the cast shown in FIG.3-(b), the particle itself is formed of different ingredients such as corpuscle, epithelium and so on, and is not constant in size nor in shape. Since for clinical identification it is necessary to depend on image itself, the judgement whether automatic classification is performed is set as "no", and the judgement of technical expert of examination is required. Although the judging condition on whether automatic classification is performed or not in FIG.3 is considered as an example, lack of necessary condition for selection other than the above determines an object as "no" in the judgement on whether automatic classification is performed or not.

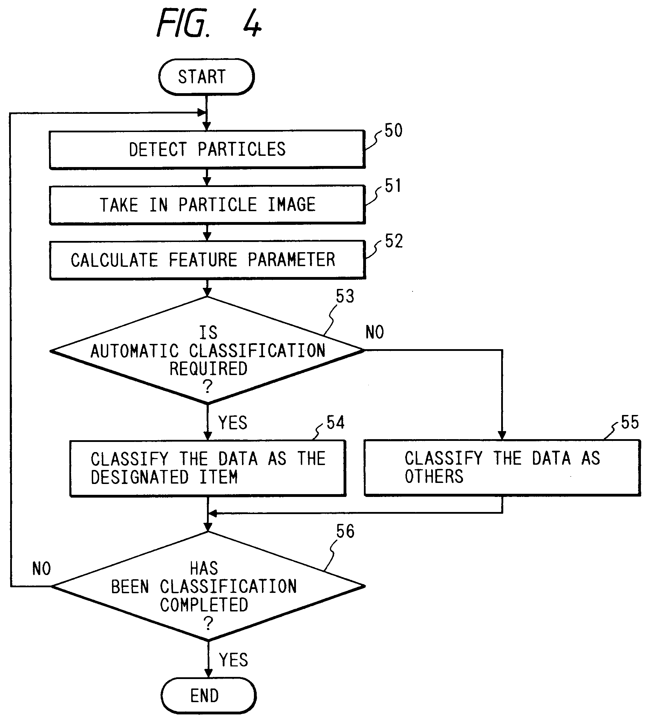

In order to explain a method to realize the present invention, a classification flow-chart is shown in FIG.4. In the embodiment in FIG.4 according to the present invention, after detecting particles in Step 50, an image of the particle is taken in and expressed with binary values in Step 51. Calculation is executed on feature parameters using the image data expressed with the binary values to perform recognition (Step 52). In Step 52, feature parameters such as diameter, length of periphery and area of the particle are calculated and further a ratio of diameter to area is calculated using these values. Judgement on whether automatic classification is required or not (Step 53) is executed according to the designated information shown in FIG.2 in each particle item. The data of the detected particles is classified as OTHERS (Step 55) if the answer in step 53 is "no" and classified as the particle item designated to need automatic classification (Step 54) if the answer in step 53 is "yes". In step 56, whether classifying has been completed or not is judged. If the answer is "yes", the flow ends and if the answer is "no", the flow returns to the first step. In the steps from Step 53 to Step 55, there are several methods considered to realize the judgement on whether automatic classification is required or not. The following are examples.

(1) The feature parameters of the particles classified as the designated particle item to be judged as YES on whether automatic classification is required or not is considered at the time of judgement (Step 53 in FIG.4). (branching method of condition)

(2) The particles are classified but are not counted as the designated particle item.
(3) The judgement is not used for a weight coefficient. (neural network method)

Methods of judging whether automatic classification is required or not from Step 53 to Step 55 will be described below in detail.

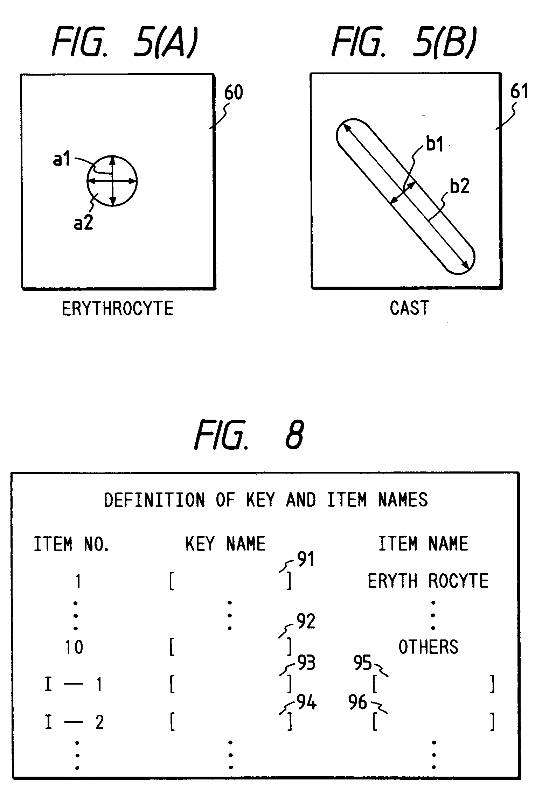

FIG.5 shows an embodiment to realize judgement on whether automatic classification is required or not without taking the feature parameters into consideration. In the particle images in FIG.5-(A) and FIG.5 (B), it is assumed that the particle image 60 is judged as an erythrocyte and the particle image 61 is judged as a cast. That is, the particle image 60 is judged as an erythrocyte since the lengths of diagonal lines a1 and a2 are nearly equal to each other. The particle image 61 is judged as a cast since the length of diagonal line b2 is n (n is lager than 1) times as large as the length of diagonal line b1. In a case where classification can be determined by the difference in the feature parameter (here, the length of diagonal line), the judgement on whether automatic classification is required or not can be realized by classifying the particles as OTHERS when the feature parameter is n times. (the item (1) in the methods of judging whether automatic classification is required or not described above). Further, by temporarily saving the results of automatic classification using these feature parameters, the results may be stored particle item by particle item according to the specification on whether automatic classification is required or not.

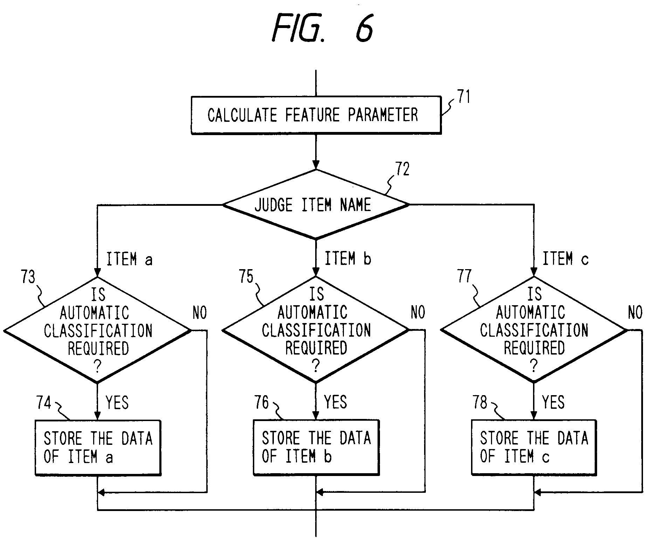

FIG.6 shows an embodiment to realize judgement on whether automatic classification is required or not according to a method where the particle is classified but is not counted as a designated particle item. A feature parameter is calculated in Step 71, and then the particle item name is judged in Step 72. Each of Steps 73, 75 and 77 is judgement for each of the particle items on whether automatic classification is required or not, and the information designated in the input area 41 in FIG.2 is used in this judgement. If judged as NO in any one of Steps 73, 75 and 77, the particle is not stored as the designated particle item for automatic classification since the process in Step 74, 76 or 78 is not executed. (the particle item (2) in the methods of judging whether automatic classification is required or not described above.)

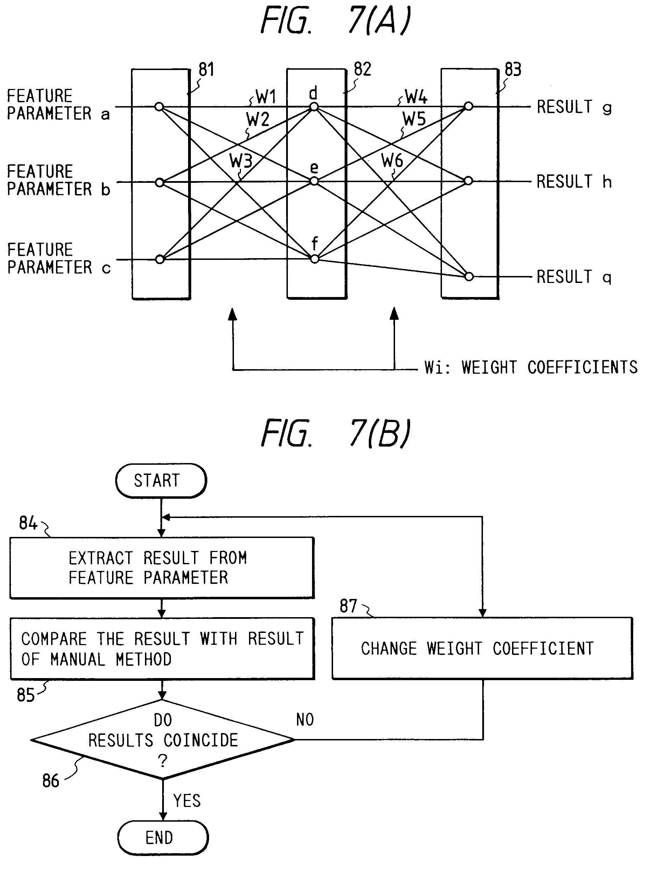

An embodiment to realize judgement on whether automatic classification is required or not according to a method where the weight coefficient is changed will be described below, referring to FIGS.7(A) and (B). FIG.7(A) shows the improvement in classification accuracy by changing the weight coefficient, and shows an example of a three-stage neural network. Feature parameters, for example, are input to an input stage 81. The feature parameters may be a diameter, length of periphery, area and so on. A middle stage 82 is provided to calculates values to be added to elements d, e and f each. For example, the value d is a sum of products of the qualities a, b and c and the weight coefficients w1, w2 and w3, respectively. And an output stage 83 is to indicates the results of classification g, h and q which may be the number corresponding to the particle item names. For example, the value g is a sum of products of the elements in the middle stage d, e and f and the weight coefficients w4, w5 and w6, respectively. The classified results are put out from the output stage 83 as values of results. Therein, the weight coefficients change the results of classification, and proper changes in the weight coefficients improve the accuracy. The weight coefficients in this embodiment are given as parameters and are rewritable. When the weight coefficients are updated, the result is extracted from the neural network in Step 85 shown in FIG.7(B). In Step 85, the obtained result is compared with a result obtained from a manual method. If the classified result is not agree with the manual result, the weight coefficients are changed. By repeating this procedure particle item by particle item, the accuracy in the classification result is improved. Further, since the item judged as NO in the judgement on whether automatic classification is required or not is not classified if the weight coefficients with concerning to the particle item, the item can be put off from the particle items to be classified automatically. Furthermore, although the updating of the weight coefficients is equivalent to the neural learning, it does not take much time and is efficient if the updating is performed only limited items.

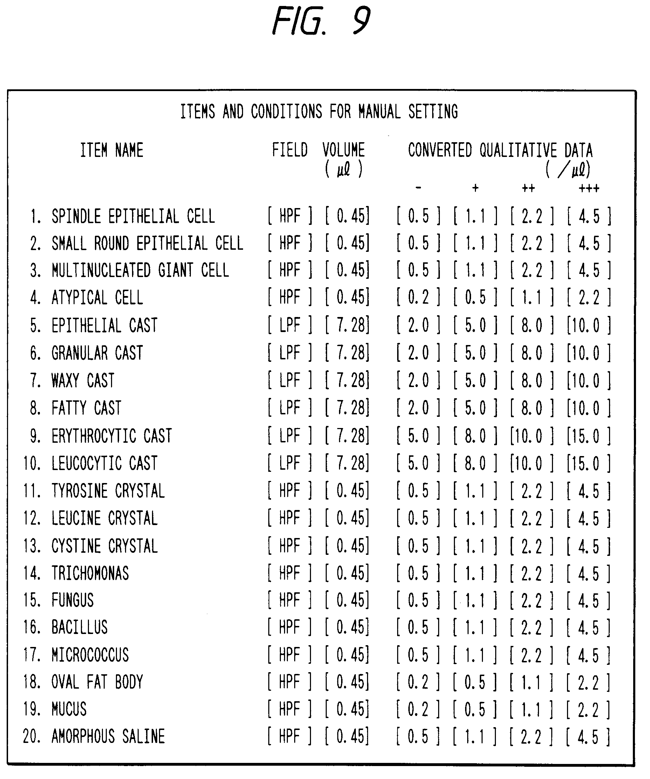

FIG.8 shows a screen to register detailed particle item names to classify the particle items automatically classified by an optical image produced by the embodiment according to the present invention. Input areas 91, 92, 93 and 94 are to interconnect between a key in the key board and a particle item, the particle item is designated by pushing the key in the key board when classifying images. Input areas 95 and 96 are areas for inputting particle item names and can be input the particle item names necessary for manual classification. By using this screen, the manual classification can be used with combining with the automatic classification.

Since capability of setting of automatic classification particle item by particle item as described above, it is possible to realize a urinary sediment examining apparatus capable of efficiently leaning, accurately classifying and efficiently classifying.

Since there are a great number of kinds of the particles in urine, it is impossible to classify all the particles automatically. There appear unclassifiable particles. Generally, only the unclassifiable particles are therefore stored to display the necessary particles through manual designation from a data inputting unit 33. However, it is possible to add particle items having a small frequency of occurrence but an important meaning for clinical judgement (for example, atypical cell, cancer cell or the like) to the object to be stored in the image memory if necessary.

The power of the TV camera is changed by switching the focusing lens 7 if necessary since in the processing described above the sizes of the particles in urine has a wide measuring range of less than 1μm in diameter to larger than 200μm.

When a technical expert or operator instructs from the data inputting unit 33 to the central processing unit 30 to display an image stored in the image memory unit 31 on the display 32 during examining by the apparatus or at an arbitrary time after examination completion, the central processing unit 30 displays the image of the particles as well as the judged result of the apparatus on the display 32.

The technical expert or operator looks at the judged result of the apparatus and the image displayed on the display 32 and corrects the result from the data input unit 33 if necessary. The central processing unit 30 executes a data calculation in the process described below using the input data and then puts out the result.

The object field of view predetermined corresponding to the particle items predetermined to need manual data correction are registered in advance by the technical expert or operator and are set on a condition setting screen shown in FIG.9. FIG.9 shows the condition setting screen concerning the particle items predetermined to need manual data correction. The letter HPF expresses a high power field of view (usually 400 times), LPF expressing a low power field of view (usually 100 times), WF expressing a whole field of view.

Letting the volume of the urinary sample measured in HPF as $V_h$, the volume of the urinary sample measured in LPF as $V_l$, the number of particles of the designated particle item i classified in HPF as $N_{hi}$, and the number of particles of the designated item i classified in LPF as $N_{li}$, the particle concentration, that is, the number of particles of the particle item i per unit volume $C_i$ can be expressed by Equation 1.

$$C_i = (N_{li} \cdot f_l / V_l) + (N_{hi} \cdot f_h / V_h),$$

where

$f_l$:　　0 in HPF, and 1 in LPF and in WF,

$f_h$:　　0 in LPF, and 1 in HPF and in WF.

The reporting form of the urinary sediment examination other than the particle concentration described above is qualitative data (-, ±, +, + +, + + + and so on) to qualitatively express the number or the particle concentration of particles in the object field of view. That is, since the volume of the urinary sample in the object field of view is known and is set in advance (or can be set by being arbitrarily input by the technical expert or operator), the number of said particles in the object field of view can be obtained by multiplying the particle concentration by the set volume of the urinary sample in the object field of view to be put out. The qualitative data is put out such a manner that which range the obtained particle concentration belongs to by inputting each of boundary values (particle concentration) of -, ±, +, + +, + + + and so on in advance.

The volume of the urinary sample in the object field of view or the boundary values may be set so as to keep the continuity of data with those in the past method.

In FIG.9, taking particle item number 1 as an example, spindle epithelial cell expresses the particle item name, HPF expressing the object field of view, 0.45 expressing the volume of the urinary sample in the object field of view. And as qualitative data conversion values, also taking particle item number 1 as an example, the symbol "-" expresses below 0.5, "+" expressing above 1.1 and below 2.2, "+ +" expressing above 2.2 and below 4.5, and "+ + +" expressing above 4.5. For the other item numbers, the same as above.

As being understood from the foregoing, by setting the object field of views registered in advance corresponding to the particle items designated by the expert or operator, it becomes possible to provide accurate examination data for the particle items which cannot be automatically classified, and consequently a more efficient and accurate urine sediment examination can be realized. Since it is possible to input data for the particle items which are unable to be automatically classified or the particle items requiring sub-classification by using the display and the input unit, the examination becomes further efficient by eliminating data inputting work using a separate apparatus. Furthermore, since the volume of the urinary sample in the object field of view or the qualitative data conversion value can be arbitrary set, the examination data having the continuity with past data can be provided.

Since it is obvious that many changes and modifications can be made in the above described details without departing from the nature and spirit of the present invention, it is to be understood that the present invention is not to be limited to the details described herein.

**Claims**

1. A urinary sediment examining apparatus comprising:

   means for passing through a predetermined position a liquid sample (13) in which particles are suspended;

   means (14~19) for detecting a particle passed through the predetermined position;

   means (33) for designating an particle item predetermined to need automatic classification; and

   means (8,24,25,26,28) for producing an image of the detected particle and determining whether the detected particle is to be classified as the designated particle item on the basis of the image.

2. A urinary sediment examining apparatus according to claim 1, further comprising means (32) for displaying names of particle item and necessity for the particle item whose name is displayed to need automatic classification.

3. A urinary sediment examining apparatus according to claim 2, wherein the displaying means is adapted to update and display the names of particle item unit by unit, where one unit is representative of the predetermined number of the names of particle item.

4. A urinary sediment examining apparatus according to claim 1, wherein the image producing and classification determining means is adapted to execute leaning so as to compare the determination of the classification with that of classification by a manual method to make them coincident with each other.

5. A urinary sediment examining apparatus comprising:

   a flow cell (4);

   means for passing through a predetermined region of the flow cell a liquid sample (13) in which urinary sedimentation particles are suspended;

   means (14~19) for detecting a urinary sedimentation particle passed through the predetermined region of the flow cell;

   means (5,7) for optically producing a still image of the detected urinary sedimentation particle;

   means (8) for converting the produced still image into electronic image data;

   means (33) for designating a particle item predetermined to need automatic classification; and

   means (26,28,29) for extracting a feature parameter of the detected urinary sedimentation particle on the basis of the converted electronic image data and determining whether the detected urinary sedimentation particle is to be classified as the designated particle item on the basis of the extracted feature parameter.

6. A urinary sediment examining apparatus according to claim 5, further comprising means (32) for displaying names of particle item and necessity for the particle item whose name is displayed to need automatic classification.

7. A urinary sediment examining apparatus comprising:

a flow cell (4);

means for passing through a predetermined position of the flow cell a liquid sample (13) in which urinary sedimentation particles are suspended;

means (14~19) for detecting urinary sedimentation particles passed through the predetermined position of the flow cell;

means (5,7,8) for optically producing still images of the detected urinary sedimentation particles and converting the produced still images into electronic image data;

means (33) for designating a particle item predetermined to need automatic classification and a particle item predetermined not to need automatic classification; and

means (28,29) for extracting feature parameters of the detected urinary sedimentation particles on the basis of the converted electronic image data and determining whether the detected urinary sedimentation particles are to be classified as the particle item predetermined to need automatic classification or the particle item predetermined not to need automatic classification on the basis of the extracted feature parameters.

8. A urinary sediment examining apparatus according to claim 3, further comprising means (32) for displaying names of particle item and necessity for the particle item whose name is displayed to need automatic classification.

9. A urinary sediment examining apparatus according to claim 8, wherein the displaying means is adapted to update and display the names of particle item unit by unit, where one unit is substantially representative of the predetermined number of the names of particle item.

10. A urine sediment examining apparatus according to claim 7, wherein the feature parameter extracting and classification determining means is adapted to classify the detected urinary sedimentation particles as either said particle item predetermined to need automatic classification or the particle item predetermined not to need automatic classification.

11. A urinary sediment examining apparatus according to claim 8, wherein the displaying means is adapted to update and display the names of particle item unit by unit, where one unit is representative of the predetermined number of the names of particle item.

12. A urinary sediment examining apparatus according to claim 7, wherein the urinary sedimentation particles determined so as to be classified as the particle item predetermined to need automatic classification and the urinary sedimentation particles determined so as to classified as the particle item predetermined not to need automatic classification are stored separately from each other.

13. A urinary sediment examining apparatus comprising:

means for passing through a predetermined position a liquid sample (13) in which particles are suspended;

means (14~19) for detecting particles passed through the predetermined position;

means (31) for registering object fields of view predetermined corresponding to particle items predetermined need manual data correction in advance;

means (33) for designating a particle item predetermined to need automatic classification and at least one of the particle items predetermined to need manual data correction; and

means (5,7,8,24,25,26,28,29) for producing images of the detected particles and determining whether the detected particles are to be classified as the designated particle item predetermined to need automatic classification on the basis of the images, the image producing and classification determining means being adapted to produce data of the detected particles classified as the designated particle item predetermined to need manual data correction, the data being for the registered object field of view corresponding to the designated particle item predetermined to need manual data correction.

14. A urinary sediment examining apparatus having means for detecting particles suspended in a urinary sample (13), means (5,7,8) for producing images of the detected particles and means (28,29) for classifying the detected particles on the basis of the images, the improvement comprising means (31) for registering object fields of view predetermined corresponding to particle items predetermined to need manual data correction in advance, and means (33) for designating at least one of the particle items, the particle classifying means being adapted to produce data of the detected particles classified as the designated particle item, the data being for the registered object field of view corresponding to the designated particle item.

15. A urinary sediment examining apparatus according to claim 14, wherein the object fields of view are selected from a high power field of view, a low power field of view or a whole field of view.

16. A urinary sediment examining apparatus according to claim 14, wherein the particle classifying means being capable of calculating the number of the detected particles per unit volume classified as the designated particle item on the basis of the object field of view corresponding to the designated particle item and a measured volume of the urinary sample.

17. A urinary sediment examining apparatus according to claim 16, further comprising means (31) for storing a volume of the urinary sample in the object field of view corresponding to the designated particle item.

18. A urinary sediment examining apparatus according to claim 16, wherein the particle classifying means being capable of converting the number of the detected particles per unit volume classified as the designated particle item into the number of the detected particles classified as the designated particle item in the object field of view corresponding to the designated particle item.

19. A urinary sediment examining apparatus according to claim 17, wherein the particle classifying means being capable of calculating the number of the detected particles classified as the designated particle item in the object field of view corresponding to the designated particle item on the basis of the number of the detected particles per unit volume classified as the designated particle item and the volume of the urinary sample in the object field of view corresponding to the designated particle item.

20. A urinary sediment examining apparatus according to any one of claim 16, wherein the particle classifying means is provided with classification ranges into which the data of the detected particles classified as the designated particle item are to be classified.

21. A urine sediment examining apparatus according to claim 20, wherein the data of the detected particles classified as the designated particle item include qualitative data representing which of the classification ranges the number of the detected particles per unit volume classified as the designated particle item is classified into.

22. A urinary sediment examining apparatus according to any one of claim 16, wherein the particle detecting means comprises a flow cell (4) through which the urinary sample is passed, the images being still images of the particles in the urinary sample produced when the particles in the urinary sample are passed through a predetermined region of the flow cell.

23. A urinary sediment examining apparatus according to claim 22, wherein the particle classifying means is adapted to classify the detected particles using neural network algorithm having self-learning capability.

FIG. 1

# FIG. 2

ITEM NAME             40

AUTOMATIC CLASSFICATION     41

# FIG. 3(A)

42

ERYTHROCYTE

# FIG. 3(B)

43

CAST

# *FIG.* 4

```
        ( START )
            │
            ▼
┌───────────────────────────┐
│     DETECT PARTICLES      │── 50
└───────────────────────────┘
            │
            ▼
┌───────────────────────────┐
│   TAKE IN PARTICLE IMAGE  │── 51
└───────────────────────────┘
            │
            ▼
┌───────────────────────────┐
│ CALCULATE FEATURE PARAMETER│── 52
└───────────────────────────┘
            │
            ▼
         ╱──────╲                    53
       ╱    IS    ╲         NO
      ╱ AUTOMATIC  ╲───────────────┐
      ╲CLASSIFICATION╱             │
       ╲ REQUIRED ╱                │
         ╲   ?  ╱                  │
            │ YES                  │
            ▼        54            ▼         55
┌───────────────────────┐  ┌───────────────────┐
│ CLASSIFY THE DATA AS  │  │ CLASSIFY THE DATA │
│ THE DESIGNATED ITEM   │  │ AS OTHERS         │
└───────────────────────┘  └───────────────────┘
            │                      │
            ◄──────────────────────┘
            ▼                  56
         ╱──────╲
  NO   ╱   HAS    ╲
◄─────╱    BEEN     ╲
      ╲CLASSIFICATION╱
       ╲ COMPLETED ╱
         ╲   ?  ╱
            │ YES
            ▼
        ( END )
```

## FIG. 5(A)

ERYTHROCYTE

## FIG. 5(B)

CAST

## FIG. 8

```
        DEFINITION OF KEY AND ITEM NAMES

ITEM NO.         KEY NAME              ITEM NAME
                              ⌐91
    1         [        ]                ERYTH ROCYTE
    .                 .                      .
    .                 .        ⌐92           .
    10        [        ]                OTHERS
                              ⌐93    95⌐
  I — 1       [        ]               [              ]
                              ⌐94    96⌐
  I — 2       [        ]               [              ]
    .                 .                      .
    .                 .                      .
```

14

# FIG. 6

## FIG. 7(A)

FEATURE PARAMETER a

FEATURE PARAMETER b

FEATURE PARAMETER c

81    82    83

W1    W2    W3    W4    W5    W6

d    e    f

RESULT g

RESULT h

RESULT q

— Wi: WEIGHT COEFFICIENTS

## FIG. 7(B)

START

84 — EXTRACT RESULT FROM FEATURE PARAMETER

COMPARE THE RESULT WITH RESULT OF MANUAL METHOD

85

86 — DO RESULTS COINCIDE ?

NO

87 — CHANGE WEIGHT COEFFICIENT

YES

END

# FIG. 9

| ITEMS AND CONDITIONS FOR MANUAL SETTING | | | | | | |
|---|---|---|---|---|---|---|
| ITEM NAME | FIELD | VOLUME ( $\mu\ell$ ) | CONVERTED QUALITATIVE DATA ( $/\mu\ell$ ) | | | |
| | | | − | + | ++ | +++ |
| 1. SPINDLE EPITHELIAL CELL | [ HPF ] | [ 0.45] | [ 0.5 ] | [ 1.1 ] | [ 2.2 ] | [ 4.5 ] |
| 2. SMALL ROUND EPITHELIAL CELL | [ HPF ] | [ 0.45] | [ 0.5 ] | [ 1.1 ] | [ 2.2 ] | [ 4.5 ] |
| 3. MULTINUCLEATED GIANT CELL | [ HPF ] | [ 0.45] | [ 0.5 ] | [ 1.1 ] | [ 2.2 ] | [ 4.5 ] |
| 4. ATYPICAL CELL | [ HPF ] | [ 0.45] | [ 0.2 ] | [ 0.5 ] | [ 1.1 ] | [ 2.2 ] |
| 5. EPITHELIAL CAST | [ LPF ] | [ 7.28] | [ 2.0 ] | [ 5.0 ] | [ 8.0 ] | [10.0 ] |
| 6. GRANULAR CAST | [ LPF ] | [ 7.28] | [ 2.0 ] | [ 5.0 ] | [ 8.0 ] | [10.0 ] |
| 7. WAXY CAST | [ LPF ] | [ 7.28] | [ 2.0 ] | [ 5.0 ] | [ 8.0 ] | [10.0 ] |
| 8. FATTY CAST | [ LPF ] | [ 7.28] | [ 2.0 ] | [ 5.0 ] | [ 8.0 ] | [10.0 ] |
| 9. ERYTHROCYTIC CAST | [ LPF ] | [ 7.28] | [ 5.0 ] | [ 8.0 ] | [10.0 ] | [15.0 ] |
| 10. LEUCOCYTIC CAST | [ LPF ] | [ 7.28] | [ 5.0 ] | [ 8.0 ] | [10.0 ] | [15.0 ] |
| 11. TYROSINE CRYSTAL | [ HPF ] | [ 0.45] | [ 0.5 ] | [ 1.1 ] | [ 2.2 ] | [ 4.5 ] |
| 12. LEUCINE CRYSTAL | [ HPF ] | [ 0.45] | [ 0.5 ] | [ 1.1 ] | [ 2.2 ] | [ 4.5 ] |
| 13. CYSTINE CRYSTAL | [ HPF ] | [ 0.45] | [ 0.5 ] | [ 1.1 ] | [ 2.2 ] | [ 4.5 ] |
| 14. TRICHOMONAS | [ HPF ] | [ 0.45] | [ 0.5 ] | [ 1.1 ] | [ 2.2 ] | [ 4.5 ] |
| 15. FUNGUS | [ HPF ] | [ 0.45] | [ 0.5 ] | [ 1.1 ] | [ 2.2 ] | [ 4.5 ] |
| 16. BACILLUS | [ HPF ] | [ 0.45] | [ 0.5 ] | [ 1.1 ] | [ 2.2 ] | [ 4.5 ] |
| 17. MICROCOCCUS | [ HPF ] | [ 0.45] | [ 0.5 ] | [ 1.1 ] | [ 2.2 ] | [ 4.5 ] |
| 18. OVAL FAT BODY | [ HPF ] | [ 0.45] | [ 0.2 ] | [ 0.5 ] | [ 1.1 ] | [ 2.2 ] |
| 19. MUCUS | [ HPF ] | [ 0.45] | [ 0.2 ] | [ 0.5 ] | [ 1.1 ] | [ 2.2 ] |
| 20. AMORPHOUS SALINE | [ HPF ] | [ 0.45] | [ 0.5 ] | [ 1.1 ] | [ 2.2 ] | [ 4.5 ] |